# EUROPEAN PATENT APPLICATION

(11) **EP 0 903 117 A2**
(43) Date of publication of application: **24.03.1999**
(21) Application number: 98117650.6
(22) Date of filing: 17.09.1998
(51) Int. Cl.: A61F 2/06, A61L 27/00

(54) **Medical materials and method for the preparation of same**

(30) Priority: 17.09.1997 JP 252313/97; 24.08.1998 JP 237657/98
(71) Applicant: TONOKURA IKA KOGYO KABUSHIKI KAISHA, Bunkyo-ku Tokyo (JP); NAICEM, LTD., Yokohama (JP)
(72) Inventor: Noishiki, Yasuharu, Yokohama (JP); Nakayama, Atsuhiko, Kanagawa-ken (JP); Tonokura, Eiji Tonokura Ika Kogyo Kabushiki Kaisha, Tokyo (JP)
(74) Representative: Laufhütte, Dieter, Dr.-Ing.

(57) **Abstract**

The medical material is configured such that support cloth of non-bioabsorptive fibers having a size of 1.0 denier or larger is tangled at random with a large number of minute non-absorptive fibers having a size of 0.5 denier or smaller. The medical material is prepared by locating an aggregate member comprised of minute fibers on support cloth; and treating said aggregate member with high-pressure fluid to cause the minute fibers to be tangled at random in a texture of the support cloth.

## Description

### CROSS-REFERENCE

The entire disclosure of Japanese Patent Applications No. 9-252,313 filed on September 17, 1997 and No. 10-237,657 filed on August 24, 1998, including specification, claims, drawings and summary is incorporated by reference in its entirety.

### BACKGROUND OF THE INVENTION

### 1. FIELD OF THE INVENTION

The present invention relates to medical materials for use as artificial blood vessels, medical patches and so on and a method for the preparation of the same.

### 2. DESCRIPTION OF THE RELATED ART

Medical materials applicable for use as artificial blood vessels, medical patches, etc are disclosed, for example, in Japanese Patent Laid-Open Publication No. 63-52,898, which are prepared by subjecting support cloth composed of minute fibers to raising treatment and then by treating the resultant raised fibers with high-pressure fluid to cause the raised fibers to be tangled in a woven texture, etc of the support cloth. Such medical materials can improve their various properties including the preventive performance from leakage of blood due to the minuteness of the minute fibers tangled in the support cloth and a low porosity based on entanglement of the minute fibers in the support cloth, as well as flexibility, resistance to raveling, ability of sticking a needle, bioadaptability (resistance to thrombosis), and the like.

Such medical materials for use as artificial blood vessels, medical patches and so on, however, suffer from the disadvantages that upon preparing the such medical materials, the support cloth should be made of minute fibers or the fibers of the support cloth should be subjected to physical or chemical treatment or other treatment to form minute fibers thereon and the resulting support cloth should be subjected to raising treatment. Therefore, medical materials having desired properties suffer from the difficulty in terms of the expedition of preparation, the number of steps for preparation, etc upon their preparation.

### SUMMARY OF THE INVENTION

Therefore, the present invention has been completed with the foregoing background taken into account and it has a primary object to provide a medical material which sustains desired properties favorable as a medical material and which can be prepared with ease and with expedition.

The second object of the present invention is to provide a method for the preparation of such medical material.

In order to achieve the first object, the present invention is basically configured as originally claimed in claim 1 to provide a medical material characterized in that support cloth made of non-bioabsorptive fibers having a size of 1.0 denier or larger as structuring fiber is tangled with minute non-bioabsorptive fibers separate and discrete from the structuring fiber of the support cloth and having a size of 0.5 denier or smaller.

Throughout the specification, the terms "tangled" and "entanglement" and their related term are intended to mean that the structuring fibers of the support cloth are twisted with smaller fibers and such smaller fibers are twisted in a texture of the support cloth, i.e. around the structuring fibers in interstices thereof, to such an extent to which the blood can pass through the texture, i.e. interstices, of the support cloth.

Preferred modes of the medical material according to the first embodiment of the present invention are configured in a manner as originally claimed in claims 2 to 13.

More specifically, in a preferred mode of the first embodiment, the medical material is configured such that the minute non-bioabsorptive fibers comprise minute fibers divided from dividable compound fibers.

In another preferred mode, the medical material is further configured such that the minute non-absorptive fibers comprise minute fibers of plural kinds having relatively different physical properties including stiffness and that the minute fibers having relatively lower physical properties are present at a rate equal to or larger than the minute fibers having relatively higher physical properties.

In a further preferred, the medical material in the preferred mode is configured such that a functional surface of the support cloth is covered with a coating layer comprised of a bioabsorptive material in a non-solubilizing state.

In a still further preferred mode of the medical material according to the embodiment is configured in such a manner that the support cloth with minute fibers tangled in a texture thereof is formed into a sheet for use as a medical patch particularly for use in reintegrating an organ of the body of an animal including the human being.

In another still further preferred mode, the medical material is configured in such a manner that the support cloth is formed into a cylinder-shaped support cloth particularly for use as an artificial blood vessel.

Further, the medical material in the another still preferred modes is configured such that the cylinder-shaped support cloth is enveloped on its outer peripheral side with a support member and further that the support member comprises an reinforcement element and an impregnation element, the reinforcement element being wound on the outer periphery of the cylinder-shaped support cloth and the impregnation element being impregnated in the support cloth and holding the minute non-absorptive fibers in the support cloth.

Furthermore, the medical material is configured such that the reinforcement element comprises a mono-filament having approximately 3,000 denier or less and preferably up to approximately 5 denier.

On the other hand, in order to achieve the second object, the present invention provides a method for the preparation of the medical materials as having the configurations as described and originally claimed in claim 14.

More specifically, the method for the preparation of the medical material according to the second embodiment of the present invention is characterized in that an aggregate member comprised of minute fibers is located on support cloth and the aggregate member is treated with high-pressure fluid to cause the minute fibers to be tangled at random in a texture of the support cloth.

Preferred modes of the method for the preparation of the medical material in the second embodiment of the present invention are configured in a manner as originally claimed in claims 15 et seq.

In a preferred mode according to the second embodiment of the present invention, the method for the preparation of the medical material is configured such that the aggregate member having a large number of minute fibers comprises an aggregate member of dividable compound fibers which are dividable into plural minute fibers,

In a more preferred mode, the method in the preferred mode is further configured that the aggregate member of the dividable compound fibers comprises non-woven cloth obtainable from the dividable compound fibers.

Further, the method for the preparation of the medical material in the more preferred mode is configured such that the minute fibers comprise minute fibers of plural kinds having relatively different physical properties including stiffness and that the minutely dividable fibers of a one kind are molten and removed from the minute fibers of plural kinds tangled in the support cloth immediately before or after the high-pressure fluid treatment of the aggregate member.

Furthermore, the method in the more preferred mode is configured in such a manner that it further comprises forming the support cloth into a cylinder-shaped support cloth; disposing a core cord in the cylinder-shaped support cloth; winding the aggregate member on an outer peripheral surface of the cylinder-shaped support cloth; and treating the aggregate member with high-pressure fluid

In a further preferred mode, the method for the preparation of the medical material in the more preferred mode is configured such that the aggregate member to be disposed on the support cloth comprises a mixture of an element of the aggregate member with adhesive liquid.

In a still further preferred mode, the method in the further preferred mode is configured such that the aggregate member is disposed on the support cloth e.g. by coating the support cloth with the adhesive liquid and attaching the adhesive liquid to the surface of the support cloth.

Still further, the method for the preparation of the medical material in the more preferred mode is configured such that the non-woven cloth has a basis weight of 300 grams per square meter or less.

In a still further preferred mode, the method for the preparation of the medical material in the more preferred mode is further configured such that the mono-filament is wound on the aggregate member wound on the outer peripheral surface of the support cloth after winding the aggregate member on the outer peripheral surface of the support cloth yet before subjecting the support cloth to the treatment with high-pressure fluid and such that the mono-filament is removed after the treatment with high-pressure fluid.

In another still further preferred modes of the second embodiment of the present invention, the method for the preparation of the medical material is configured such that the mono-filament is of a core-sheath structure in which a sheath portion has a melting point lower than a melting point of the core portion while the melting point of the core portion is substantially equal to melting point of the minute fibers and the sheath portion of the mono-filament is molten by heating treatment into molten liquid after the treatment with high-pressure fluid and the resulting molten liquid is caused to permeate the support cloth.

Further, in another still further preferred mode, the method for the preparation of the medical material is configured in such a manner that the sheath portion of the mono-filament and the minute fibers on the support cloth are made each of polypropylene.

Other objects, features and advantages will become apparent in the course of the following description with reference to the accompanying drawings.

### BRIEF DESCRIPTION OF THE DRAWINGS

Figure 1 is an enlarged partial schematic perspective view showing a dividable compound fibers.
Figure 2 is a perspective view showing a non-woven cloth prepared from the dividable compound fibers, which is used for the preparation of a medical patch.
Figure 3 is a perspective view for describing high-pressure treatment upon preparing the medical patch.
Figure 4 is a perspective view showing a medical patch as an end product.
Figure 5 is an enlarged perspective view showing an artificial blood vessel in accordance with an embodiment of the present invention.
Figure 6 is an enlarged sectional view showing a mono-filament of a core-sheath configuration as a support member.
Figure 7 is an enlarged illustration showing a state in which non-woven cloth and the mono-filaments are wound on a cylindrical support cloth.
Figure 8 is an enlarged illustration for describing a state of the support cloth after the high-pressure treatment.

### DESCRIPTION OF THE PREFERRED EMBODIMENTS

The present invention will be described in more detail by way of embodiments with reference to the accompanying drawings.

A medical material according to an embodiment of the present invention may be employed specifically in a sheet form for a medical patch, etc or in a cylindrical form for an artificial blood vessel e.g. for use in reintegrating an organ of an animal including an organ of the human being. The medical material is common among specific modes in a basic configuration of the first embodiment in which support cloth comprised of non-bioabsorptive fibers are tangled with minute fibers comprised of non-bioabsorptive fibers which are separate and discrete in material from the fibers of the support cloth.

A description of the medical material will now be made of a medical patch according to an aspect of the first embodiment of the present invention.

The medical patch for use in this aspect of the embodiment may has a basic configuration such that support cloth in a sheet form is tangled with minutely divided fibers divided from dividable compound fibers.

As the support cloth, there may be employed any cloth in the form of a sheet including, for example, knitted cloth, woven cloth, plaited cords, etc. Further, the support cloth is woven, etc. so as to have a coarse texture, etc suitable for use as a medical material.

The fibers for use with the support cloth may preferably include, for example, polyester, polyamide, polytetrafluoroethylene, polyolefin, polyethylene terephthalate, or the like, although the kind is not limited to a particular one. Also, a degree of fineness of a mono-filament of such fibers is not limited to a particular one and it may range specifically from approximately 0.8 denier to 100 denier, preferably 1 denier or larger.

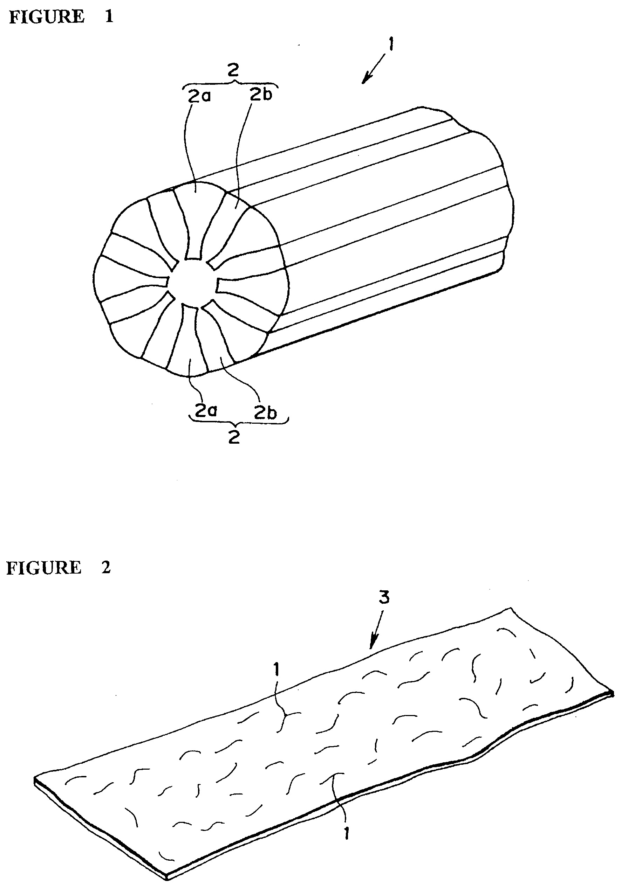

The specific configuration of the medical patch will be described in more detail with reference to Figure 1. As shown in Figure 1, the dividable compound fibers 1 comprise strings of threads consisting of a plurality (e.g. 16 strings in this aspect of the embodiment) of minutely dividable fibers (minute fibers) 2 which are disposed so as to become next to each other one by one in the peripheral direction of the dividable compound fibers 1 in order to allow each of the minutely divided fibers 2 to be divided and dispersed by applying a predetermined degree of an impact force to the dividable compound fibers.

The dividable compound fibers 1 may be configured so as to have a size of filament of approximately 1 denier or larger, preferably approximately 3 denier and a length to the axial center of approximately 50 mm in this specific mode of the embodiment. Therefore, when the dividable compound fibers 1 are divided into 16 divisions comprised of the minutely divided fibers 2 as in this specific mode, each of the minutely divided fibers 2 may become as minute in fineness as approximately 0.5 denier or smaller, preferably smaller up to approximately 0.2 denier. It is to be noted herein, however, that the size of the minutely dividable fibers may vary with the size of the dividable compound fibers and a number of divisions comprised of minute fibers and that a smaller size of the minutely dividable fibers is more preferred as long as they can achieve the features sought to be gained by this invention.

For the minutely divided fibers 2 originating from the dividable compound fibers 1, in this specific mode of the embodiment, there may preferably be employed dividable compound fibers comprised of two different kinds of polymers which are less compatible with each other. As shown in Figure 1, minutely dividable fibers 2a of a one kind and minutely dividable fibers 2b of the other kind may be disposed adjacent to each other alternately in the peripheral direction of the dividable compound fibers 1. In a specific mode, for instance, polypropylene resin fiber may be employed for the minutely dividable fibers 2a while polyethylene terephthalate resin fiber may be employed for the minutely dividable fibers 2b. It is to be noted herein, however, that the kind of fibers constituting the dividable compound fibers is not limited to two kinds and may vary e.g. with properties sought to be achieved by the medical material of this invention.

The state of entanglement of the support cloth with the minutely divided fibers 2 of the dividable compound fibers 1 may be such that the support cloth is tangled with the minutely divided fibers 2 and the minutely divided fibers 2 are tangled in a woven texture, i.e. interstices, etc of the support cloth so as to allow the texture to become narrower, that is, to become smaller in size or lower in porosity yet to be large enough to allow the blood to pass through the texture of the support cloth. This configuration can improve various properties of the medical material, including, e.g. stiffness, flexibility, the preventive performance from blood leakage, resistance to raveling, ability of sticking a needle, bioadaptability (resistance to thrombosis), etc, as compared with the case where the support cloth is composed of minute fibers only or where the support cloth is tangled with fibers raised by conventional raising treatment.

A description will now turn to a process for the preparation of the medical patch according to a aspect of the first embodiment of the present invention.

As shown in Figure 2, the dividable compound fibers 1 are first shaped into non-woven cloth 3 in a sheet form by conventional process. The medical material in a sheet form may permit easy handling for tangling the support cloth with the minutely divided fibers 2 of the dividable compound fibers 1. The non-woven cloth 3 may be prepared by conventional card M/C, high-pressure treatment, or the like, singly or combined. Further, as the non-woven cloth 3, they may be employed, e.g. non-woven cloth comprised of the dividable compound fibers 1 themselves or non-woven cloth comprised of the minutely divided fibers 2 separated or divided from the dividable compound fibers 1. The non-woven cloth 3 may preferably have a basis weight of 300 grams per square meter or less, although any kind of non-woven cloth can also be employed as long as it does not exert any adverse influence upon the effects sought to be achieved by the present invention.

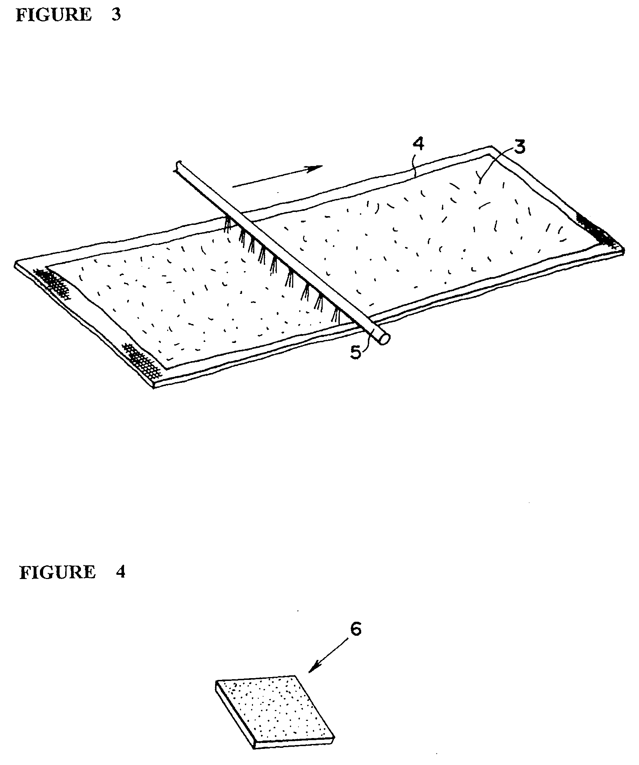

Next, as shown in Figure 3, the non-woven cloth 3 is disposed on the support cloth 4, followed by subjecting the non-woven cloth 3 to high-pressure treatment from top to cause the support cloth 4 to be tangled with the minutely divided fibers 2 divided from the dividable compound fibers 1. Particularly, when non-woven cloth comprised of the dividable compound fibers 1 themselves is employed upon this treatment, the high-pressure treatment allows the minutely dividable fibers 2 to be divided and separated from the dividable compound fibers 1 as well as the minutely divided fibers 2 to be tangled as minute fibers in the support cloth.

In this mode of the embodiment of the present invention, the high-pressure treatment may comprise e.g. jetting out water streams against the non-woven cloth 3 downward from plural jet outlets of a water discharge tube 5 to effect water jet punch treatment to cause the minute fibers 2 of the dividable compound fibers 1 to be tangled and twisted in a texture of the support cloth 4. The force of jetting out water streams may be set from the viewpoint of tangling the support cloth 4 with the minutely divided fibers 2 to an appropriate extent. More specifically, it may be set so as to make it strong to break the dividable fibers of the dividable compound fibers 1 into minute fibers 2 by applying jet pressure to the dividable compound fibers 1 and to tangle and twist the minute fibers 2 in the support cloth 4.

Thereafter, as needed, the support cloth 4 may be coated on its functional surface or its applicable surface with a bioabsorptive material which may be selected singly or plurally from e.g. collagen, gelatin, albumin, chitin, cellulose, fibroin, alginic acid, heparin, heparan sulfate, hyaluronic acid, dermatan acid, a derivative thereof and so on. The support cloth 4 is then cut into a given size to yield a medical patch 6 as shown in Figure 4.

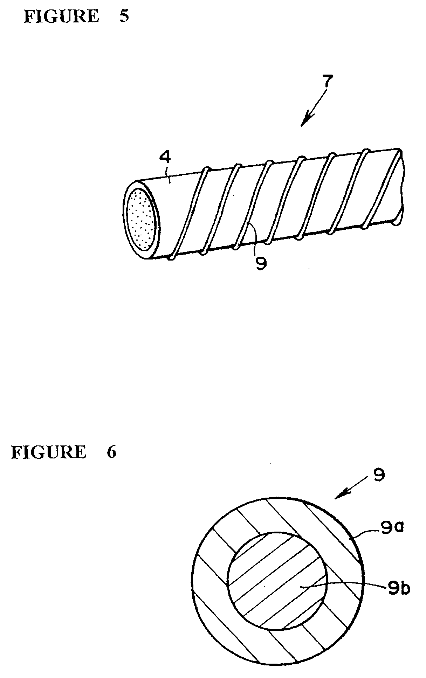

Then, a description will be made of an artificial blood vessel according to another aspect of the first embodiment of the invention with reference to Figure 5.

As shown in Figure 5, an artificial blood vessel 7 in accordance with another aspect of the first embodiment of the present invention may comprise a basic configuration in which a cylindrical support cloth 4 is tangled with the minutely divided fibers 2 of the dividable compound fibers 1 and a mono-filament 9 is wound on the outer peripheral surface of the cylindrical support cloth 4 in a spiral way.

The support cloth 4 for use as artificial blood vessels may have substantially the same configuration as the support cloth in a sheet form suitable for use as the medical patch 6 as described above. It may comprise knitted cloth, woven cloth, plaited cords, etc. Likewise, the dividable compound fibers 1 for use as the artificial blood vessels may have substantially the same configuration as the sheet-formed support cloth suitable for use as the medical patch as described above.

The mono-filament 9 to be wound on the support cloth 4 may have a size in the range of approximately 3,000 denier or smaller, e.g. smaller up to approximately 3 denier, and may be in a core-sheath configuration, as shown in Figure 6. The mono-filament 9 may be configured in such a way that a melting point of a sheath portion 9a is set to be lower than that of a core portion 9b and that the melting point of the core portion 9b is set so as to be nearly equal to a melting point of the structuring fibers of the minutely divided fibers 2. In this aspect of the embodiment, the mono-filament 9 may be made of a material that is the same as the minutely divided fibers 2a of a one kind (e.g. polypropylene resin fiber yet the material of the sheath portion has a melting point different from that of the material of the core portion).

The mono-filament 9 may be wound on the support cloth 4 at an appropriate pitch which may be set to be, e.g. 2 mm.

The sheath portion 9a of the mono-filament 9 may be molten by thermal treatment and the support cloth 4 is impregnated with the resulting molten liquid which is then allowed to coagulate in the support cloth 4, thereby forming a coagulated material in the support cloth 4. The coagulation of the molten material of the mono-filament 9 in the support cloth 4 can permit reinforcement of the support cloth 4 in a cylindrical form in association with the core portion 9b of the mono-filament 9. Furthermore, the coagulated material formed in the support cloth 4 can envelop a portion of the minute fibers 2 tangled in the texture of the support cloth 4 and hold it therein to thereby enhance reinforcement of the entanglement of the minute fibers 2.

The artificial blood vessel 7 may be prepared by steps of a process as will be described hereinafter.

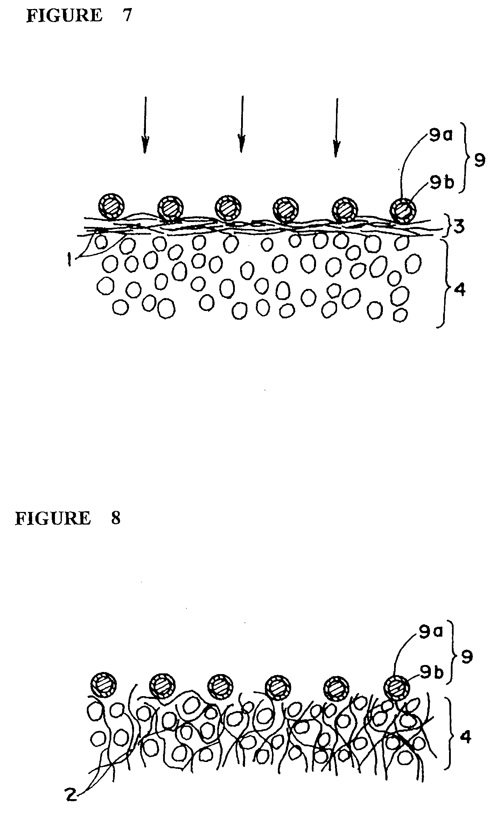

First, the support cloth 4 is formed in a cylindrical shape and a core cord is inserted in or engaged with the cylindrical support cloth 4. Then, as shown in Figure 7, the non-woven cloth 3 comprised of the dividable compound fibers 1 is wound on the outer peripheral surface of the cylindrical support cloth 4 and the mono-filament 9 is further wound on the non-woven cloth 3, followed by treating the non-woven cloth 3 with high-pressure fluid in an appropriate manner to cause the minute fibers 2 of the dividable compound fibers 1 to be tangled and twisted in the support cloth 4.

Then, the support cloth 4 on which the non-woven cloth 3 and the mono-filament 9 are wound is subjected to treatment with high-pressure fluid in a manner as will be indicated in Figure 7. The high-pressure fluid treatment may extrude the minute fibers 2 divided from the dividable compound fibers 1 through the cylindrical support cloth 4 toward the inner peripheral side (the bottom side in Figure 7) of the cylindrical support cloth 4 in order to tangle the support cloth 4 with the minutely divided fibers 2, as referred to in Figure 8. In this case, when the non-woven cloth 3 is comprised of the dividable compound fibers 1 themselves, the minutely divided fibers 2 are divided from the dividable compound fibers 1 by the treatment with high-pressure fluid and at the same time the support cloth 4 is tangled with the minutely divided fibers 2.

Further, in this instance, it is preferred that the cylindrical support cloth 4 is turned inside out after the high-pressure treatment on the outer side thereof and the currently outside surface is then subjected to the high-pressure treatment. This treatment can tangle the both sides of the support cloth 4 with the minutely divided fibers 2, thereby ensuring a further reinforcement of the cylindrical support cloth 4.

Then, the support cloth 4 is then processed thermally after the treatment with high-pressure fluid. More specifically, the sheath portion 9a of the mono-filament 9 is molten to allow the support cloth 4 to be tangled with the resulting molten liquid. Upon coagulation of the molten liquid, the coagulated material can tightly hold a portion (an upper portion in Figure 8) of the minutely divided fibers 2 with which the support cloth 4 is tangled.

When the sheath portion 9a of the mono-filament 9 is molten by the thermal treatment at this time, the core portion 9b thereof can be sustained in the state in which it is wound spirally on the outer peripheral side of the support cloth 4 without being molten because the melting point of the sheath portion 9a is set to be lower than the melting point of the core portion 9b.

The thermal treatment for the support cloth 4 after the high-pressure treatment may be carried out with an oven, laser beams or by other means.

The artificial blood vessel 7 can be prepared in the manner as described above. Further, as needed, the artificial blood vessel 7 may be prepared by coating its inner peripheral surface of the support cloth 4 with a coating material such as a bioabsorptive material including e.g. gelatin, albumin, collagen, etc. in the manner as described above.

It is to be understood herein as a matter of course that although the present invention is described by taking the medical patche and artificial blood vessel as specific examples of the medical material, the present invention can be applied to any possible usage of the such material.

The present invention will be described in more detail by way of examples with reference to the accompanying drawings.

### Example 1: Medical Patch

Card having a basis weight of 30 grams per square meter was prepared by passing raw cotton of dividable compound fibers 1 (3 denier, filament length: 45mm) through card M/C, which was formed so as to be dividable into 16 divisions of minutely divided fibers 2 consisting of polyethylene terephthalate (50%) and polypropylene (50%). The resulting card was placed on plain woven support cloth of polyethylene terephthalate and subjected to water jet punch treatment through a nozzle having a discharge outlet diameter of 0.1 mm and a discharge outlet distance of 1.0 mm at pressure of 50 kg per square centimeter and 80 kg per square centimeter at two stages to cause the support cloth 4 to be tangled with the minutely divided fibers 2 divided from the compound fibers 1.

It is found as a result of observation that the resulting medical patch is configured in such a manner that the support cloth of polyethylene terephthalate is tangled in its woven texture with a large number of minutely fibers of polyethylene terephthalate and polypropylene divided from the dividable compound fibers.

In order to measure the intensity of resistance to raveling the minutely divided fibers from the support cloth, the medical patch was cut with scissors at 45° with respect to the direction of the water jet punch treatment and a suture was sewn in the position apart by 3 mm from the cut end section and pulled toward the cut end section, As a result, it was found that the intensity of resistance to raveling was 1,079 grams when translated into weight.

### Example 2: Medical Patch

The support cloth was subjected to water jet punch treatment in two stages at the pressure of 50 kg per square centimeter and 120 kg per square centimeter under substantially the same manner as in Example 1. The resulting support cloth was found to have an intensity of resistance to raveling of 1,079 grams when translated into weight.

### Example 3: Medical Patch

Raw cotton of the dividable compound fibers 1 (3 denier; filament length: 45 mm) prepared so as to allow the minutely dividable fibers 2 comprised of polyethylene terephthalate (50%) and polypropylene (50%) to be divided into 16 divisions was passed through card M/C and then subjected to water jet punch treatment five times at the maximum water pressure of 70 kg per square centimeter yielding non-woven cloth having a basis weight of 35 grams per square meter. The non-woven cloth was placed on plain woven support cloth of polyethylene terephthalate and subjected to water jet punch treatment at the discharge outlet size of 0.1 mm and the discharge outlet distance of 1.0 mm in two stages at the water pressure of 50 kg per square centimeter and 80 kg per square centimeter to cause the minutely divided fibers to be tangled in the support cloth.

It is confirmed as in Example 1 that the resulting medical patch has a large number of minutely divided polyethylene terephthalate and polypropylene fibers tangled in a woven texture of the support cloth of polyethylene terephthalate and that the intensity of resistance to raveling was measured to be 952 grams when translated into weight.

### Example 4: Medical Patch

The support cloth was subjected to water jet punch treatment in two stages at the pressure rates of 50 kg per square centimeter and 120 kg per square centimeter under substantially the same manner as in Example 3. The resulting support cloth was found to have an intensity of resistance to raveling of 1,155 grams as translated into weight.

### Example 5: Medical Patch

Raw cotton of dividable compound fibers 1 (3 denier; filament length: 45 mm) prepared so as to allow minutely dividable fibers 2 comprised of polyethylene terephthalate (50%) ad polypropylene (50%) to be divided into 16 divisions was passed through card M/C to yield non-woven cloth having a basis weight of 35 grams per square meter. The non-woven cloth was placed on plain woven support cloth of polyethylene terephthalate and then subjected to water jet punch treatment at the discharge outlet size of 0.1 mm and the discharge outlet distance of 1.0 mm in two stages at the water pressure of 50 kg per square centimeter and 80 kg per square centimeter to cause the minutely divided fibers to be tangled in the support cloth.

It is observed that the resulting medical patch has a large number of minutely divided polyethylene terephthalate and polypropylene fibers tangled in a woven texture of the support cloth of polyethylene terephthalate.

Thereafter, n-decane was added to the support cloth treated above and the resulting support cloth was molten by heating at the temperature of 150° C for 2 hours to melt out and remove polypropylene only.

The stiffness and softness at this time was measured by JIS (Japanese Industrial Standards) L 10966.19.1 A method (45° cantilever method) and the value was found to be 37% in the longitudinal direction (advancement direction of the water jet punch treatment) with respect to the support cloth with no polypropylene removed by melting and 48% in the latitudinal direction (parallel to the water jet punch treatment) with respect thereto.

### Comparative Example 1: Medical Patch

Raw cotton of dividable compound fibers 1 (3 denier; filament length: 45 mm) prepared so as to allow minutely divided fibers 2 comprised of polyethylene terephthalate (50%) and polypropylene (50%) to be divided into 16 divisions was passed through card M/C and then subjected to water jet punch treatment five times at the discharge outlet size of 0.1 mm, the discharge outlet distance of 1.0 mm and the water pressure of 70 kg per square centimeter yielding non-woven cloth having a basis weight of 35 grams per square meter. The intensity of resistance to raveling was found to be 142 grams when translated into weight.

### Example 6: Artificial Blood Vessel

Raw cotton of dividable compound fibers 1 (3 denier; filament length: 45 mm) prepared so as to allow minutely divided fibers 2 comprised of polyethylene terephthalate (50%) and polypropylene (50%) to be divided into 16 divisions was passed through card M/C yielding non-woven cloth having a basis weight of 35 grams per square meter. The non-woven cloth was placed on the plain woven support cloth of polyethylene terephthalate fibers formed in a cylindrical shape with a core cord engaged therein and subjected to water jet punch treatment at the water pressure of 50 kg per square centimeter and 80 kg per square centimeter while rotating under given conditions to thereby cause the minutely divided fibers to be tangled in the cylindrical support cloth.

It was observed that the resulting artificial blood vessel has a large number of minutely divided polyethylene terephthalate and polypropylene fibers tangled in the woven texture of the support cloth of polyethylene terephthalate.

Thereafter, in order to observe the intensity of resistance to raveling, the artificial blood vessel was cut at 45° in the direction of the water jet punch treatment with scissors and a suture was sewn through the artificial blood vessel in the position apart by 3 mm from the cut end, followed by pulling the suture toward the cut end. As a result, the intensity of resistance to raveling was found to be 1,011 grams when translated into weight.

### Example 7: Artificial Blood Vessel

Raw cotton of dividable compound fibers 1 (3 denier; filament length: 45 mm) prepared so as to allow minutely divided fibers 2 comprised of polyethylene terephthalate (50%) and polypropylene (50%) to be divided into 16 divisions was passed through card M/C yielding non-woven cloth having a basis weight of 35 grams per square meter. The non-woven cloth was placed on plain woven support cloth of polyethylene terephthalate formed in a cylindrical shape with a core cord engaged therein, and polypropylene (300 denier) for structuring a core-sheath configuration (a melting point of the core portion: 165°C; a melting point of the sheath portion: 138°C) was wound on the support cloth at the angle of 60° with respect to the axial center of the cylindrical support cloth. Thereafter, the support cloth was subjected to water jet punch treatment at the pressure of 50 kg per square centimeter and 120 kg per square centimeter while rotating it under given conditions to thereby allow the cylindrical support cloth to be tangled with the minutely divided fibers.

Thereafter, the support cloth was placed in an oven at the temperature of about 140°C for 10 minutes. Upon heating treatment, a portion of polypropylene derived from the non-woven cloth with the minute fibers tangled in the support cloth of polyethylene terephthalate was held by the polypropylene sheath portion structuring the outer support member and the core polypropylene portion is secured intact to the outer peripheral portion of the support cloth as a support member.

The artificial blood vessel prepared in the manner as described above is configured such that the sheath polypropylene (coagulated material) portion of the polypropylene support member is formed as if roots develop into the inside from the outer peripheral surface of the support cloth. In other words, it is observed that the minutely divided fibers (polypropylene) are held on the support cloth and the minutely divided fibers are held on the support cloth in a more secured way than the support cloth that has not been processed by heating. The intensity of resistance to raveling was measured to be 1,234 grams when translated into weight.

### Example 8: Artificial Blood Vessel

Raw cotton of dividable compound fibers 1 (3 denier; filament length: 45 mm) prepared so as to allow minutely divided fibers 2 comprised of polyethylene terephthalate (50%) and polypropylene (50%) to be divided into 16 sections was passed through card M/C yielding a non-woven cloth having a basis weight of 35 grams per square meter. The non-woven cloth was placed in a n-decane solvent and heated at 150°C for 2 hours to melt out and remove only polypropylene completely (this was confirmed by infrared analysis).

On the other hand, a tubular support cloth (having an outer diameter of 8.3 mm and an inner diameter of 8.0 mm) of a knitted texture was prepared from polyester (polyethylene terephthalate) fibers of 50 denier and a core cord was engaged in the tubular support cloth and the non-woven cloth comprised of polyester alone with the polypropylene molten out and removed was wound on the tubular support cloth in a two-ply manner (Sample 1), in a four-ply manner (Sample 2) and in a six-ply manner (Sample 3), followed by jetting out high-pressure water streams against the support cloth from a nozzle having a discharge outlet size of 0.25 mm and a discharge outlet distance of 2.5 mm at the pressure rates of 30 kg per square centimeter for one time and 100 kg per square centimeter for five times. This treatment causes the non-woven cloth to be tangled in the texture of the support cloth and thereby forms an artificial blood vessel of a configuration in which finely divided fibers (approximately 0.2 denier or smaller) are tangled in the knitted texture of the support cloth.

The artificial blood vessel so formed was then measured for a water penetration rate in accordance with the U.S. Standards, i.e. ANSI/AMMI VP20-1994. The results of measurement reveal that Sample 1, Sample 2 and Sample 3 demonstrated the water penetration rates of 5,836 ml/cm²/min, 1,980 ml/cm²/min and 1,035 ml/cm²/min, respectively. These results indicate that the water penetration rate of the artificial blood vessel can be controlled by varying the rate of entanglement of minutely divided fibers. Example 9: Artificial Blood Vessel

The artificial blood vessel prepared in Example 8 was cut in an oblique direction at 45° with respect to the lengthwise axis and a 2-0 suture was sewn thereon in the position apart by 1 mm from the cut section in order to measure the extent of raveling fibers. As a result, it was found that Sample 1, Sample 2 and Sample 3 had the intensity of raveling fibers at 0.968 kgf, 1.074 kgf and 1.117 kgf, respectively. For the test, measuring instrument (Model: EZ-TEST-500N; Shimadzu, Japan) was used.

For comparative purposes, the intensity of raveling fibers was measured under the identical conditions for the following matters: the tubular support cloth only, an artificial blood vessel product commercially available from company A (having a water penetration rate of 2,293 ml/cm²/min), an artificial blood vessel product commercially available from company B (having a water penetration rate of 2,409 ml/cm²/min), and an artificial blood vessel commercially available from company C (having a water penetration rate of 1,820 ml/cm²/min). As a result, they indicated the intensity of raveling fibers at 0.286 kgf, 0.665 kgf, 0.662 kgf, and 0.791 kgf, respectively. For those measurements, there were used the commercially available artificial blood vessels each having an outer diameter of 8.5 mm and an inner diameter of 8.0 mm.

### Example 10: Artificial Blood Vessel

Sample 1 of the artificial blood vessel prepared in Example 8 was subjected to a burst test in accordance with the U.S. Standards (ANSI/AMMI VP20-1994). AT the same time, the extent of the maximal deformation up to bursting was measured. For comparative purposes, the materials used for comparative purposes in Example 9 were also used. For the burst test, measuring instrument (Model: EZ-TEST-500N; Shimadzu, Japan) was used.

The results of the burst test reveal that Sample 1, the support cloth, the commercially available product of company A, the commercially available product of company B, and the commercially available product of company C had the load of 23.519 kgf, 20.521 kgf, 10.450 kgf, 17.848 kgf, and 20.821 kgf, respectively. Further, the maximal deformation at the respective load was 5.08 mm, 5.21 mm, 9.83 mm, 7.25 mm, and 8.66 mm.

It is found from the above burst test results that the artificial blood vessel with the minutely divided fibers tangled therein demonstrated the highest strength against bursting with the water penetration ratio taken into consideration, compared with the commercially available artificial blood vessels, and at the same time that it exhibited the smallest extent of maximal deformation. In other words, the artificial blood vessel with the minutely divided fibers tangled therein has the configuration that is stronger and less deformed as compared with the commercially available artificial blood vessels. For the burst test, measuring instrument (Model: EZ-TEST-500N; Shimadzu, Japan) was used.

### Example 11: Artificial Blood Vessel

The artificial blood vessel of Sample 1 was connected to a portion of an artificial heart-lung circuit and a filter of 30 micron was disposed so as to form a closed circuit which in turn was filled with purified water. Then, the purified water was circulated through the closed circuit at 20° C and at the pulse stream rate of 2,000 ml per minute for 12 hours continuously. The purified water was used for this test to increase the higher freedom of movement of minute fibers in order to allow the test to be conducted under more severe experimental conditions than the use of physiological saline or serum.

After the circulation for 12 hours, the filter was taken out from the circuit and observed with a high resolution stereomicroscope. As a result of microscopic observation, it was found that no minute fibers was discovered.

### EFFECTS OF THE INVENTION

The medical material according to the first embodiment in an aspect of the present invention has the basic configuration in which the support cloth of non-absorptive fibers having a size of 1.0 denier or larger is tangled at random with a large number of minute non-absorptive fibers having a size of 0.5 denier or smaller, which are separate and discrete from the structuring fibers of the support cloth. Therefore, the medical material presents the advantages that, even if the support cloth itself has a coarse texture, the minute non-absorptive fibers tangled, such a texture, i.e. interstices etc of the support cloth can make a texture of the medical material fine while sustaining flexibility and other properties inherent in the support cloth itself, thereby enabling a prevention of blood from leakage from the medical material. Further, the medical material according to this invention is provided with improved properties, e.g. resistance to raveling minute fibers from support cloth, a high ability of sticking a needle, bioadaptability (resistance to thrombosis), etc, thereby ensuring high performance sought to be achieved as artificial blood vessels, medical patches, etc.

Particularly, in this instance, there are employed the minute non-absorptive fibers having an average size of 0.5 denier or less. If there are employed minute non-absorptive fibers having an average size larger than approximately 0.5 denier to the contrary, the resulting medical material becomes less compatible with host tissues, thereby decreasing the effectiveness of the medical material sought to be achieved by causing the minute fibers to be entangled in the support cloth and reducing the effectiveness sought to be gained by causing the host tissues to penetrate therein and to propagate them therein. Therefore, the medical material according to the present invention can ensure the desired functions that are sought to be provided by the medical materials.

On the other hand, the medical material according to the present invention is configured such that the support cloth is tangled in its interstices with the minute non-absorptive fibers separate and discrete from the structuring fibers of the support cloth and the resulting medical material can be prepared simply by tangling the minute non-absorptive fibers in the support cloth by treatment with high-pressure fluid. Therefore, it is not required to make the support cloth itself from minute fibers and then to subject the support cloth to raising treatment. Moreover, as the medical material is configured such that the support cloth acts as a base and the minute non-absorptive fibers supplement the action of the support cloth, there may be employed a variety of general use support cloth made of non-absorptive fibers as a material for the medical material according to the present invention, thereby reducing costs of preparing support cloth which amount to a large ratio of the costs of preparing the medical material. Further, the medical material according to the present invention can be prepared by simple processes with ease and with expedition.

As the medical material according to the present invention is further of a configuration such that the support cloth is tangled with the minute non-absorptive fibers separate and discrete from the structuring fibers of the support cloth, the rate of the non-absorptive fibers with respect to the support cloth can be selected with high freedom and this is of great help in preparing different types of medical materials that can be adapted to versatile usage including medical patch, artificial blood vessel, etc.

Furthermore, the medical material according to the present invention can control its deformation and improve its physical strength by varying the extent of entanglement of the support cloth with the minute fibers separate and discrete from the structuring fibers of the support cloth.

In a specific mode of the embodiment, the present invention provides the medical material in which the minute non-absorptive fibers comprise minute fibers divided from dividable compound fibers which in turn are separate and discrete from the structuring fibers of the support cloth and are disposed on the support cloth so as to divide the minute fibers therefrom by treatment with high-pressure fluid and to disperse them in the support cloth. At the same time, the minute fibers divided from the dividable compound fibers can be entangled in a texture, i.e. interstices etc of the support cloth. Therefore, the medical material according to the present invention can achieve the effects as gained by the basic configuration of the medical material of this invention as well as it requires neither the step of mechanically tangling the minute fibers in the support cloth nor the step of mechanically forming the minute fibers on the support cloth, thereby leading to the easy and expeditious preparation of the medical material.

In a more specific mode, the medical material according to the embodiment of the present invention is further configured in such a manner that the minute non-absorptive fibers further comprise minute fibers of plural kinds having relatively different physical properties including stiffness and the support cloth is tangled with the minute fibers of plural kinds in such a manner that the minute fibers having relatively lower physical properties are present at a rate equal to or larger than the minute fibers having relatively higher physical properties. Therefore, this configuration of the medical material can provide medical materials having more flexible and softer properties, in addition to the effects to be achieved by the basic configuration of the medical material of this invention, thereby enabling improvements in handling performance and functionality as medical materials for use in artificial blood vessels, medical patches, etc.

Further, in a more specific mode, the present invention provides the medical material which is further configured such that the functional surface of the support cloth is covered with a coating comprised of a bioabsorptive material in a non-solubilizing state, so that leakage of blood from artificial blood vessels can be prevented in association with the characteristics of the material forming the coating layer and compatibility with tissues of a host using the medical patches, artificial blood vessels, etc can be improved. Moreover, the coating layer can also be tangled closely with the minute fibers tangled in the support cloth so that the anchoring effect (i.e. the effect for fixing the minute fibers to the support cloth) can also be improved to a remarkable extent as compared with conventional medical materials in which fibers of general size are used.

Moreover, the present invention provides the medical material in which the minute non-absorptive fibers comprising the minute fibers of plural kinds having relatively different physical properties are configured in the manner as described above and in which the functional surface of the support cloth is covered with a coating comprised of a bioabsorptive material in a non-solubilizing state. Therefore, this mode of the present invention can achieve the effects as can be achieved by the above modes of the medical material in which minute fibers divided from the dividable compound fibers are used as the minute non-absorptive fibers. In other words, this mode of the medical material can prevent the leakage of blood from the artificial blood vessels and improve compatibility with the living tissues of the host by taking advantage of the characteristics of the material forming the coating layer. Moreover, as the minute fibers are entangled in the support cloth, the coating layer can also be entangled closely in the minute fibers, thereby assisting remarkable improvements in the anchoring effects (i.e. fixing effects) compared with the use of fibers of general size.

In another specific mode, where the medical material according to the present invention is further configured, in addition to the basic configuration, in such a way that the minute fibers of plural kinds having relatively different physical properties including stiffness are used as the minute non-absorptive fibers in the manner as defined above, the medical material can additionally achieve higher flexibility and elasticity. This can also improve handling performance and functionality of the medical material as medical materials for use as artificial blood vessels, medical patches, etc.

In a specific mode in which the medical material is additionally configured by covering the functional surface of the support cloth with the coating layer comprised of a bioabsorptive material in a non-solubilizing state, the medical material can achieve the effects to be gained by the coating layer, in addition to the effects to be gained by the medical material in the another specific mode as described above, i.e. to the effects of preventing the leakage of blood and enhancing compatibility with the living tissues, etc. Further, as the coating layer can also closely tangle the minute fibers which in turn are tangled in the support cloth, it can improve the anchoring effects (i.e. fixing effects) to a remarkable extent, as compared with conventional medical materials prepared with fibers of general size.

In a specific mode where the medical material is configured such that the support cloth of the non-absorptive fibers having a size of 1.0 denier or larger is tangled at random with a large number of the separate and discrete, minute non-absorptive fibers having a size of 0.5 denier or smaller and the functional surface of the support cloth is covered with the coating layer comprised of the bioabsorptive material in a non-solubilizing state, the medical material can achieve the effects to be gained by the coating layer, i.e. improvements in the anchoring effect (i.e. fixing effect), in addition to the effects to be gained by the medical material in the basic configuration of the medical as described above.

In a still further mode where the medical material is formed in a sheet shape appropriate for use as a medical patch for reintegrating damaged tissues of the organ of the living body, the medical material can provide a medical patch particularly superior in high handling performance and bioadaptability.

Likewise, in a still specific mode of the medical material which is formed in a cylinder shape appropriate for use as an artificial blood vessel, the medical material can provide an artificial blood vessel particularly superior in high handling performance and bioadaptability.

In a more specific mode of the medical material formed into a cylinder-shaped member is enclosed at its outer peripheral side with the support member, the resulting artificial blood vessel can be reinforced by the support member, in addition to improved handling performance and bioadaptability.

Further, in a more specific mode of the medical material in which the support member comprises the reinforcement element wound on the outer periphery of the cylinder-shaped member and the impregnation element impregnated in the support cloth to hold the minute non-absorptive fibers in the support cloth, the resulting medical material presents the advantages that the artificial blood vessel can ensure an improved reinforcement by means of the reinforcement element and a sure prevention of separation of the minute fibers from the support cloth by the support with the impregnation element as well as the entanglement of the minute fibers in the support cloth.

Moreover, in a more specific mode, the medical material is configured by the reinforcement element which comprises the mono-filament of 3,000 denier or less so that the resulting medical material can sustain elasticity as well as flexibility, in addition to performing the effects as achieved by the specific mode where the support member comprises the reinforcement element and the impregnation element.

In accordance with the second embodiment of the present invention as described above, the method for the preparation of the medical material has a basic configuration in which an aggregate member comprised of a large number of minute fibers is located on support cloth and the aggregate member is treated with high-pressure fluid to cause the minutely divided fibers to be tangled at random in a texture, i.e. interstices, etc of the support cloth. This high-pressure fluid treatment can cause the minute fibers to be tangled in the support cloth effectively and efficiently without forming the support cloth with minute fibers themselves by weaving the aggregate member with the minute fibers themselves or subjecting the aggregate member by physical or chemical treatment or subjecting the support cloth to raising treatment. Therefore, the method in the basic configuration can form the medical material having the above-mentioned features and advantages with ease and with expedition and at low costs.

In a specific mode of the present invention, the method for the preparation of the medical material comprising a large number of the minute fibers is configured such that the aggregate member comprises an aggregate of dividable compound fibers which are dividable into a large number of minute fibers. Thus, the dividable compound fibers can be divided into and disperse the minute fibers structuring the dividable compound fibers by the treatment with high-pressure fluid. At the same time, likewise, the high-pressure fluid treatment can tangle the minute fibers in the support cloth so that the method according to the present invention does not additionally and discretely require the step for mechanically tangling the minute fibers in the support cloth and the step of forming the support cloth with the such minute fibers. Therefore, the method in the above configuration can provide the medical material having the above-mentioned features and advantages with ease and with expedition as well as at low costs.

Further, in a specific mode in which the method according to the present invention uses the non-woven cloth obtained from the dividable compound fibers as the aggregate member of the dividable compound fibers, this method can improve handling performance including disposition of the aggregate member, adjustment of the amount of the aggregate member, etc.

Moreover, in a specific mode, the method according to the present invention is further configured such that the minute fibers are comprised of the minute fibers of plural kinds having relatively different physical properties and the minutely divided fibers of a kind out of the minute fibers of plural kinds tangled in the support cloth are molten out and removed immediately before or after treating the aggregate member with high-pressure fluid, this method can adjust the rate of the minute fibers of different physical properties in the support cloth, thereby yielding the medical material having a given rate of the minute fibers of desired physical properties and leading to the preparation of the medical material having good handling performance as described above.

Furthermore, in another specific mode, the method further comprises forming the support cloth into a cylinder-shaped support cloth; disposing a core cord in the cylinder-shaped support cloth; winding the aggregate member on a outer peripheral surface of the cylinder-shaped support cloth; and treating the aggregate member with high-pressure fluid. Therefore, this method can provide artificial blood vessels having the above-mentioned features and advantages with ease and with expedition and at low costs.

In another specific mode, the method is further configured in such a manner that the aggregate member to be disposed on the support cloth comprises a mixture of an element of the aggregate member with an adhesive liquid. Therefore, this method can expedite and improve workability upon preparation of the medical materials according to the present invention because the aggregate member can be held on the support cloth by taking advantage of the adhesive liquid.

Further, in another specific mode, the method is configured such that the aggregate member is disposed on the support cloth, e.g. by coating a surface of the support cloth with the adhesive liquid and attaching the element of the aggregate member to the adhesive liquid. Therefore, likewise, this method can expedite and improve workability upon preparation of the medical materials according to the present invention because the aggregate member can be held on the support cloth by taking advantage of the adhesive liquid.

In a still further specific mode where the method uses the non-woven cloth having a basis weight of 300 grams per square meter or less, this method can achieve the effects and advantages as can be achieved by the specific modes of the method where the non-woven cloth obtained by the dividable compound fibers is used as the aggregate member. Therefore, this method allows the minutely divided minute fibers to be tangled in the support cloth in a desired state, thereby capable of ensuring high functions e.g. handling performance and bioadaptability as expected to be achieved as medical materials including e.g. medical patches and artificial blood vessels.

Further, in a still further specific mode, the method is configured such that the minute fibers are comprised of plural kinds having relatively different physical properties and the method further comprises melting out and removing the minute fibers of a kind out of the minute fibers of plural kinds tangled in the support cloth immediately before or after treating the aggregate member with high-pressure fluid. Therefore, this method can also achieve the effects as described above, including adjustment of the rate of the minute fibers having different physical properties, leading to a well-balanced adjustment of the desired properties of the medical material according to the present invention.

In a still further specific mode, the method is further configured by locating the non-woven cloth obtainable from the dividable compound fibers as the aggregate member on the support cloth; forming said support cloth into a cylinder-shaped support cloth; disposing the core cord in the cylinder-shaped support cloth; winding the aggregate member on an outer peripheral surface of the cylinder-shaped support cloth; and treating the aggregate member with high-pressure fluid. Therefore, this method can effectively and efficiently provide the artificial blood vessel having the features and advantages as described above..

In a still further specific mode, the method is further configured such that the minute fibers of plural kinds having relatively different physical properties are used and that the minutely divided fibers of a kind out of the minute fibers of plural kinds tangled in the support cloth are molten out and removed immediately before or after treating the aggregate member with high-pressure fluid. Therefore, this method permits the appropriate adjustment of the minute fibers of such a certain kind, thereby producing the artificial blood vessels having the features and advantages as described above with ease and with expedition.

In another still specific mode, the method is further configured by forming the support cloth into a cylinder-shaped support cloth from the non-woven cloth having the basis weight of 300 grams per square meter or less; disposing the core cord in the cylinder-shaped support cloth; winding the aggregate member on the outer peripheral surface of the cylinder-shaped support cloth; and treating the aggregate member with high-pressure fluid. Therefore, this method can efficiently and readily provide the medical material having the features and advantages as described above.

Moreover, the method is configured such that the minute fibers of plural kinds having relatively different physical properties are tangled at random in the support cloth and that the minutely divided fibers of a kind out of the minute fibers of plural kinds tangled in the support cloth are molten out and removed immediately before or after treating the aggregate member with high-pressure fluid. Therefore, the method can provide the artificial blood vessels having the above described features and advantages with ease and with expedition.

In addition, the method is further configured by forming the support cloth into a cylinder-shaped support cloth; disposing the core cord in the cylinder-shaped support cloth; winding the aggregate member on an outer peripheral surface of the cylinder-shaped support cloth; and treating the aggregate member with high-pressure fluid. Therefore, this method can produce the artificial blood vessels having the features and advantages as described above with ease and with expedition.

In a still further mode, the method is likewise configured such that the support cloth is formed in a cylindrical shape; the core cord is disposed in the cylinder-shaped support cloth; the aggregate member is wound on the outer peripheral surface of the cylinder-shaped support cloth on which the aggregate member comprised of the minute fibers is placed; and the aggregate member is treated with high-pressure fluid. Therefore, this method can produce the artificial blood vessels having the features and advantages as described above with ease and with expedition.

In a still further mode, the method is configured such that the aggregate member located on the support cloth comprises a mixture of the element of the aggregate member with the adhesive liquid. Therefore, this specific mode of the method can hold the aggregate member on the support cloth by means of the adhesive liquid, thereby improving workability of the preparation of the artificial blood vessels.

In a still further specific mode, the method is configured in such a way that the aggregate member comprised of minute fibers is disposed on the support cloth by coating the surface of the support cloth with the adhesive liquid and attaching the element of the aggregate member to the adhesive liquid and that the aggregate member is then treated with high-pressure fluid to cause the minute fibers to be tangled at random in a texture of the support cloth. Therefore, in this specific case, too, this method can hold the aggregate member closely on the support cloth by means of the adhesive liquid, thereby improving workability of the preparation of the artificial blood vessels

Further, another specific mode of this embodiment of the present invention is configured such that the mono-filament is wound on the aggregate member disposed on the outer peripheral surface of the support cloth after winding the aggregate member on the outer peripheral surface of the cylinder-shaped support cloth yet before treating the aggregate member with high-pressure fluid. Therefore, this method allows the mono-filament to hold the aggregate member tightly on the outer peripheral surface of the support cloth and the minute fibers to be tangled in the support cloth in a desired fashion.

Moreover, in another specific mode, the method is configured such that the mono-filament is removed after treating the aggregate member with high-pressure fluid. Therefore, this method can enhance the freedom of selecting materials for the mono-filament because the mono-filament is removed after the high-pressure fluid treatment and the mono-filament is not left intact in the product.

Furthermore, this method uses the mono-filament of a core-sheath structure in which the sheath portion has a melting point lower than the melting point of the core portion while the melting point of the core portion is substantially equal to a melting point of the minute fibers and the sheath portion of the mono-filament is molten into molten liquid by heating treatment to penetrate the molten liquid into the texture of the support cloth. Therefore, this specific mode of the method can hold the aggregate member in the cylinder-shaped support cloth by means of the mono-filament in an appropriate fashion during the manufacturing processes, particularly during the high-pressure fluid treatment. Moreover, when the artificial blood vessels are prepared from the cylinder-shaped support cloth as the product, the core portion of the mono-filament assists in reinforcing the resulting artificial blood vessels, while the molten liquid soaked in the texture of the support cloth and coagulated therein envelopes part of the minute fibers tangled in the support cloth, thereby ensureing a sure prevention of the minute fibers from being separated from the support cloth.

In addition, the method uses polypropylene for the sheath portion of the mono-filament and for the minute fibers to be tangled in the support cloth. Therefore, the mono-filament makes its molten liquid obtained from its sheath portion more compatible with the minute fibers when the molten liquid coagulates, thereby ensuring a sure attachment of the minute fibers to the support cloth.

## Claims

1. A medical material wherein support cloth of non-bioabsorptive fibers having a size of 1.0 denier or larger are tangled at random with minute non-bioabsorptive fibers having a size of 0.5 denier or smaller.

2. A medical material as claimed in claim 1, wherein said minute non-absorptive fibers comprise minute fibers divided from dividable compound fibers.

3. A medical material as claimed in claim 2, wherein;
said minute non-absorptive fibers comprise minute fibers of plural kinds having relatively different stiffness; and
said support cloth is tangled with said minute fibers of plural kinds in such a manner that minute fibers having relatively lower stiffness are present at a rate equal to or larger than minute fibers having relatively higher stiffness.

4. A medical material as claimed in claim 2, wherein a functional surface of said support cloth is covered with a coating layer comprised of a bioabsorptive material in a non-solubilizing state.

5. A medical material as claimed in claim 3, wherein a functional surface of said support cloth is covered with a coating layer comprised of a bioabsorptive material in a non-solubilizing state.

6. A medical material as claimed in claim 1, wherein;
said minute non-absorptive fibers comprise minute fibers of plural kinds having relatively different stiffness; and
said support cloth is tangled with said minute fibers of plural kinds in such a manner that minute fibers having relatively lower stiffness are present at a rate equal to or larger than minute fibers having relatively higher stiffness.

7. A medical material as claimed in claim 6, wherein a functional surface of said support cloth is covered with a coating layer comprised of a bioabsorptive material in a non-solubilizing state.

8. A medical material as claimed in claim 1, wherein a functional surface of said support cloth is covered with a coating layer comprised of a bioabsorptive material in a non-solubilizing state.

9. A medical material as claimed in claim 2, wherein the medical material is of a sheet form for use as a medical patch for reintegrating an organ of the body of an animal.

10. A medical material as claimed in claim 2, wherein the medical material is of a cylindrical form for use as an artificial blood vessel.

11. A medical material as claimed in claim 10, wherein said medical material of a cylindrical form is enclosed at an outer peripheral surface thereof with a support member.

12. A medical material as claimed in claim 11, wherein said support member comprises an reinforcement element and an impregnation element; said reinforcement element being wound on an outer periphery of said medical material of a cylindrical form and said impregnation element being impregnated in said support cloth and holding said minute non-absorptive fibers in said support cloth.

13. A medical material as claimed in claim 12, wherein said reinforcement element comprises a mono-filament of 3,000 denier or less.

14. A method for the preparation of a medical material, comprising:
locating an aggregate member comprised of minute fibers on support cloth; and
treating said aggregate member with high-pressure fluid to cause said minute fibers to be tangled at random in a texture of said support cloth.

15. A method as claimed in claim 14, wherein said aggregate member comprises an aggregate of dividable compound fibers which are dividable into minute fibers.

16. A method as claimed in claim 15, wherein said aggregate member comprises non-woven cloth obtainable from said dividable compound fibers.

17. A method as claimed in claim 15, wherein said minute fibers are minute fibers of plural kinds having relatively different physical properties;
further comprising melting out and removing minutely divided fibers of a kind out of said minute fibers of plural kinds tangled in said support cloth immediately before or after treating said aggregate member with high-pressure fluid.

18. A method as claimed in claim 15, further comprising:
forming said support cloth into a cylinder-shaped support cloth;
disposing a core cord in said cylinder-shaped support cloth;
winding said aggregate member on an outer peripheral surface of said cylinder-shaped support cloth; and
treating said aggregate member with high-pressure fluid.

19. A method as claimed in claim 15, wherein:
said aggregate member to be disposed on said support cloth comprises a mixture of an element of said aggregate member with an adhesive liquid.

20. A method as claimed in claim 15, wherein disposition of said aggregate member on said support cloth comprises coating a surface of said support cloth with said adhesive liquid and attaching said element of said aggregate member to said adhesive liquid.

21. A method as claimed in claim 16, wherein said non-woven cloth has a basis weight of 300 grams per square meter or less.

22. A method as claimed in claim 16, wherein said minute fibers are minute fibers of plural kinds having relatively different physical properties;
further comprising melting out and removing minutely divided fibers of a kind out of said minute fibers of plural kinds tangled in said support cloth immediately before or after treating said aggregate member with high-pressure fluid.

23. A method as claimed in claim 16, further comprising:
forming said support cloth into a cylinder-shaped support cloth;
disposing a core cord in said cylinder-shaped support cloth;
winding said aggregate member on an outer peripheral surface of said cylinder-shaped support cloth; and
treating said aggregate member with high-pressure fluid.

24. A method as claimed in claim 21, wherein said minute fibers are minute fibers of plural kinds having relatively different physical properties;
further comprising melting out and removing minutely divided fibers of a kind out of said minute fibers of plural kinds tangled in said support cloth immediately before or after treating said aggregate member with high-pressure fluid.

25. A method as claimed in claim 21, further comprising:
forming said support cloth into a cylinder-shaped support cloth;
disposing a core cord in said cylinder-shaped support cloth;
winding said aggregate member on an outer peripheral surface of said cylinder-shaped support cloth; and
treating said aggregate member with high-pressure fluid.

26. A method as claimed in claim 14, wherein said minute fibers are minute fibers of plural kinds having relatively different physical properties;
further comprising melting out and removing minutely divided fibers of a kind out of said minute fibers of plural kinds tangled in said support cloth immediately before or after treating said aggregate member with high-pressure fluid.

27. A method as claimed in claim 26, further comprising:
forming said support cloth into a cylinder-shaped support cloth;
disposing a core cord in said cylinder-shaped support cloth;
winding said aggregate member on an outer peripheral surface of said cylinder-shaped support cloth; and
treating said aggregate member with high-pressure fluid.

28. A method as claimed in claim 14, further comprising:
forming said support cloth into a cylinder-shaped support cloth;
disposing a core cord in said cylinder-shaped support cloth;
winding said aggregate member on an outer peripheral surface of said cylinder-shaped support cloth; and
treating said aggregate member with high-pressure fluid.

29. A method as claimed in claim 14, wherein:
said aggregate member to be disposed on said support cloth comprises a mixture of an element of said aggregate member with an adhesive liquid.

30. A method as claimed in claim 14, wherein disposition of said aggregate member on said support cloth comprises coating a surface of said support cloth with said adhesive liquid and attaching said element of said aggregate member to said adhesive liquid.

31. A method as claimed in claim 18, wherein a mono-filament is wound on said aggregate member disposed on an outer peripheral surface of said support cloth after winding said aggregate member on the outer peripheral surface of said cylinder-shaped support cloth yet before treating said aggregate member with high-pressure fluid.

32. A method as claimed in claim 31, further comprising removing said mono-filament after treating said aggregate member with high-pressure fluid.

33. A method as claimed in claim 31, wherein said mono-filament is of a core-sheath configuration in which a sheath portion has a melting point lower than a melting point of a core portion and the melting point of said core portion is substantially equal to a melting point of said minute fibers;
further comprising forming molten liquid by melting said sheath portion of said mono-filament by heating treatment; and
impregnating said support cloth with said molten liquid.

34. A method as claimed in claim 33, wherein said sheath portion of said mono-filament and said minute fibers on said support cloth are made each of polypropylene.
